# EUROPEAN PATENT SPECIFICATION

(11) **EP 3 390 482 B1**
(45) Date of publication and mention of the grant of the patent: **03.03.2021**
(21) Application number: 16822357.6
(22) Date of filing: 16.12.2016
(51) Int. Cl.: C08G 18/48, C08G 18/62, C08G 18/76, C08G 18/20, C08G 18/32, C08G 18/40

(54) **POLYISOBUTYLENE-POLYURETHANES AND MEDICAL DEVICES CONTAINING THE SAME**
POLYISOBUTYLEN-POLYURETHANE UND MEDIZINPRODUKTE DAMIT
POLYISOBUTYLÈNE-POLYURÉTHANES ET DISPOSITIFS MÉDICAUX LES CONTENANT

(30) Priority: 17.12.2015 US 201562268732 P
(43) Date of publication of application: 24.10.2018
(73) Proprietor: Cardiac Pacemakers, Inc., St. Paul, MN 55112-5798 (US)
(72) Inventor: DELANEY, JR., Joseph T., Minneapolis Minnesota 55416 (US); GURUNG, Niraj, Sauk Rapids Minnesota 55379 (US); WILLOUGHBY, Patrick, Shoreview Minnesota 55126 (US); WULFMAN, David R., Minneapolis Minnesota 55405 (US); ADENUSI, Adegbola O., Burnsville Minnesota 55306 (US); AREMU, Adeniyi O., Brooklyn Park Minnesota 55444 (US)
(74) Representative: Pfenning, Meinig & Partner mbB
(86) International application number: PCT/US2016/067363
(87) International publication number: WO 2017/106774

(56) References cited:
- US-A- 4 939 184
- US-A- 6 087 454
- US-A1- 2011 152 989

## Description

### CROSS-REFERENCE TO RELATED APPLICATION

This application claims priority to Provisional Application No. 62/268,732, filed December 17, 2015.

### TECHNICAL FIELD

The present invention relates to polymeric materials. More specifically, the invention relates to polyisobutylene-polyurethane block copolymers, methods for making polyisobutylene-polyurethane block copolymers, and medical devices containing polyisobutylene-polyurethane block copolymers.

### BACKGROUND

Polymeric materials are widely used in the field of medical devices. For example, polymeric materials such as silicone rubber, polyurethane, and fluoropolymers are used as coating and/or insulating materials for medical leads, stents, catheters, and other devices.

Block copolymers are polymeric materials made of alternating sections of polymerized monomers. Polyisobutylene-polyurethane block copolymers are polymeric materials with many unique physical and mechanical properties. Exemplary properties, which may be particularly desirable in the field of medical devices, include thermal stability, chemical resistance, biocompatibility, and gas impermeability, among others.

### SUMMARY

The invention is defined in the appended claims. Disclosed is a polymeric material including a polyisobutylene-polyurethane block copolymer and a tertiary amine catalyst. The polyisobutylene-polyurethane block copolymer includes soft segments including at least one polyisobutylene diol residue, and hard segments including at least one diisocyanate residue. The tertiary amine catalyst includes 2,6-dimethylpyridine.

### DETAILED DESCRIPTION

A more complete understanding of the present invention is available by reference to the following detailed description of numerous aspects and embodiments of the invention. The detailed description of the invention which follows is intended to illustrate but not limit the invention.

In accordance with various aspects of the disclosure, polyisobutylene-polyurethane block copolymers (also referred to herein collectively as "PIB-PUR") and methods for making the same are disclosed. Medical devices that can be implantable or insertable into the body of a patient and that comprise at least one polyisobutylene urethane copolymer are also disclosed. PIB-PUR is a thermoplastic polyurethane (TPUs) that contains hard and soft segments. PIB-PUR is useful in a number of applications, including in medical devices used for insertion or implantation into a patient because they are hydrolytically stable and have good oxidative stability.

Polyurethanes are a family of copolymers that are synthesized from polyfunctional isocyanates (e.g., diisocyanates, including both aliphatic and aromatic diisocyanates) and polyols (e.g., macroglycols). Commonly employed macroglycols include polyester diols, polyether diols and polycarbonate diols. The macroglycols can form polymeric segments of the polyurethane. Aliphatic or aromatic diols may also be employed as chain extenders, for example, to impart improved physical properties to the polyurethane.

In some embodiments, the polyisobutylene urethane copolymer includes one or more polyisobutylene segments, one or more segments that includes one or more diisocyanate residues, optionally one or more additional polymeric segments (other than polyisobutylene segments), and optionally one or more chain extenders.

As used herein, a "polymeric segment" or "segment" is a portion of a polymer. The polyisobutylene segments of the polyisobutylene urethane copolymers are generally considered to constitute soft segments, while the segments containing the diisocyanate residues are generally considered to constitute hard segments. The additional polymeric segments may include soft or hard polymeric segments. As used herein, soft and hard segments are relative terms to describe the properties of polymer materials containing such segments. Without limiting the foregoing, a soft segment may display a glass transition temperature (Tg) that is below body temperature, more typically from 35 °C to 20 °C to 0 °C to -25 °C to -50 °C or below. A hard segment may display a Tg that is above body temperature, more typically from 40 °C to 50 °C to 75 °C to 100 °C or above. Tg can be measured by differential scanning calorimetry (DSC), dynamic mechanical analysis (DMA) and/or thermomechanical analysis (TMA).

The weight ratio of soft segments to hard segments in the polyisobutylene urethane copolymers of the various embodiments can be varied to achieve a wide range of physical and mechanical properties, and to achieve an array of desirable functional performance. For example, the weight ratio of soft segments to hard segments in the polymer can be varied from 99:1 to 95:5 to 90:10 to 75:25 to 50:50 to 25:75 to 10:90 to 5:95 to 1:99, more particularly from 95:5 to 90:10 to 80:20 to 70:30 to 65:35 to 60:40 to 50:50, and even more particularly, from about 80:20 to about 50:50. In some embodiments, the soft segment components can be about 40% to about 70% by weight of the copolymer, and the hard segment components can be about 30% to about 60% by weight of the copolymer.

In some embodiments, the copolymer may include polyisobutylene in an amount of about 60% to about 100% by weight of the soft segments and a polyether, a polyester, or a polycarbonate in an amount of about 0% to about 40% by weight of the soft segments. For example, the copolymer may include soft segments in an amount of about 40% to about 70% by weight of the copolymer, of which polyisobutylene is present in an amount of about 60% to about 100% by weight of the soft segments and polyether is present in an amount of about 0% to about 40% by weight of the soft segments. In another embodiment, the copolymer may include soft segments in an amount of about 40% to about 70% by weight of the copolymer, of which polyisobutylene (e.g., a polyisobutylene diol residue) is present in an amount of about 70% to about 95% by weight of the soft segments and a polyether (e.g., polytetramethylene oxide diol residue) is present in an amount of about 5% to about 40% by weight of the soft segments.

Diisocyanates for use in forming PIB-PUR of the various embodiments include aromatic and non-aromatic (e.g., aliphatic) diisocyanates. Aromatic diisocyanates may be selected from suitable members of the following, among others: 4,4'-methylenediphenyl diisocyanate (MDI), 2,4'-methylenediphenyl diisocyanate (2,4-MDI), 2,4- and/or 2,6-toluene diisocyanate (TDI), 1,5-naphthalene diisocyanate (NDI), para-phenylene diisocyanate, 3,3'-tolidene-4,4'-diisocyanate and 3,3'-dimethyl-diphenylmethane-4,4'-diisocyanate. Non-aromatic diisocyanates may be selected from suitable members of the following, among others: 1,6-hexamethylene diisocyanate (HDI), 4,4'-dicyclohexylmethane diisocyanate (H12-MDI), 3-isocyanatomethyl-3,5,5-trimethylcyclohexyl isocyanate (isophorone diisocyanate or IPDI), cyclohexyl diisocyanate, and 2,2,4-trimethyl-1,6-hexamethylene diisocyanate (TMDI).

In some embodiments, a polyether diol such as polytetramethylene oxide diol (PTMO diol), polyhexametheylene oxide diol (PHMO diol), polyoctamethylene oxide diol or polydecamethylene oxide diol, can be combined with a polyisobutylene diol and diisocyanate to form a polyisobutylene polyurethane copolymer. In some embodiments, PIB-PUR may have a generally uniform distribution of polyurethane hard segments, polyisobutylene segments and polyether segments to achieve favorable micro-phase separation in the polymer. In some embodiments, polyether segments may improve key mechanical properties such as Shore hardness, tensile strength, tensile modulus, flexural modulus, elongation tear strength, flex fatigue, tensile creep, and/or abrasion performance, among others.

PIB-PUR in accordance with the various embodiments may further include one or more optional chain extender residues. Chain extenders can increase the hard segment length, which can in turn results in a copolymer with a higher tensile modulus, lower elongation at break and/or increased strength.

Chain extenders can be formed from aliphatic or aromatic diols, in which case a urethane bond is formed upon reaction with an isocyanate group. Chain extenders may be selected from suitable members of the following, among others: 1,4 cyclohexanedimethanol, alpha,omega-alkane diols such as ethylene glycol (1,2-ethane diol), 1,4-butanediol (BDO), and 1,6-hexanediol. Chain extenders may be also selected from suitable members of, among others, short chain diol polymers (e.g., alpha,omega-dihydroxy-terminated polymers having a molecular weight less than or equal to 1000) based on hard and soft polymeric segments (more typically soft polymeric segments) such as those described above, including short chain polyisobutylene diols, short chain polyether polyols such as polytetramethylene oxide diols, short chain polysiloxane diols such as polydimethylsiloxane diols, short chain polycarbonate diols such as polyhexamethylene carbonate diols, short chain poly(fluorinated ether) diols, short chain polyester diols, short chain polyacrylate diols, short chain polymethacrylate diols, and short chain poly(vinyl aromatic) diols. Chain extenders may also be selected form suitable glycols, such as propylene glycol, dipropylene glycol, and tripropylene glycol.

A polymeric material including PIB-PUR can be made by mixing soft segment components, including polyisobutylene diol, and hard segment components, including a diisocyanate, together and heating the mixture to an elevated temperature. An elevated temperature is any temperature above room temperature, such as 30°C, 40°C, 50°C, 60°C, 70°C, 80°C, 90°C, or 100°C, or any temperature between any of the preceding temperatures.

The polyisobutylene diol may be a telechelic polyisobutylene diol formed from by carbocationic polymerization beginning with a difunctional initiator compound, such as 5-*tert*-butyl-1,3-*bis*(1-methoxy-1-methylethyl)benzene (hindered dicumyl ether), end-capped with hydroxyl functional groups and prepared by means known in the art (see, *e.g.,* Ivan, B. and J.P. Kennedy, Living carbocationic polymerization. XXX. One-pot synthesis of allyl-terminated linear and tri-arm star polyisobutylenes, and epoxy- and hydroxy-telechelics therefrom. J. Polym. Sci., Part A: Polym. Chem., 1990. 28(1): p. 89-104). The resulting compound may be a polyisobutylene diol according to the formula:

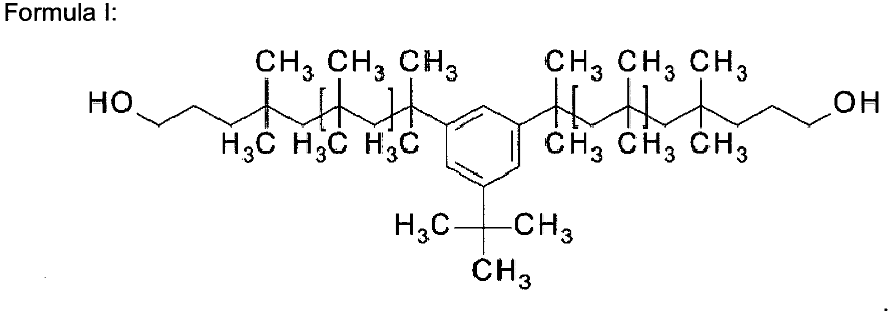

The diisocyanate includes two isocyanate groups, as shown the example of 4,4'-methylenediphenyl diisocyanate according to the formula:

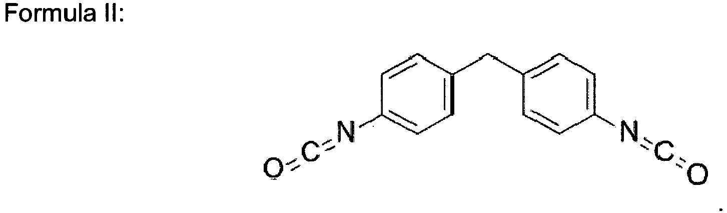

Each of the isocyanate groups may react with a hydroxyl group of the polyisobutylene diol, or with any other diol, such as polytetramethylene oxide diol or 1,4 butanediol to form a urethane linkage. As this polymerization process continues, PIB-PUR is formed. An exemplary PIB-PUR including polyisobutylene diol residue, diisocyanate residue in the form of 4,4'-methylenediphenyl diisocyanate residue, chain extender residue in the form of 1,4-butanediol residue, and polyether diol residue in the form of polytetramethylene oxide diol residue, is shown by the formula:

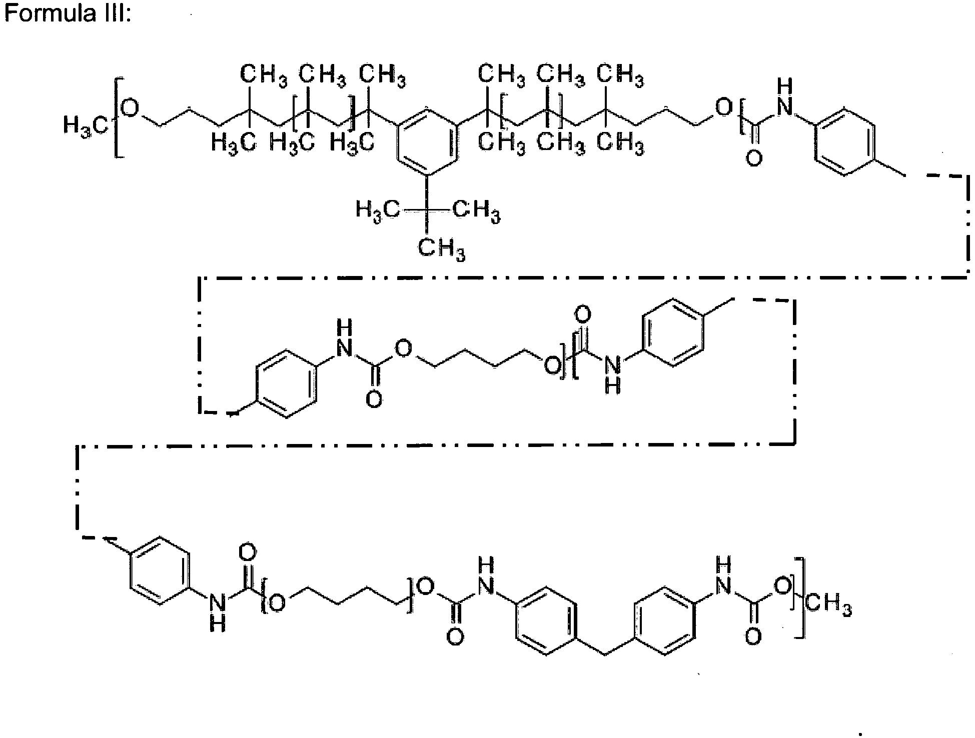

A catalyst added to the mixture can increase the reaction rate and significantly reduce the time for substantial completion of the reaction. However, many known polyurethane catalysts have not been shown to be suitable for catalyzing a reaction with polyisobutylene diol. Polyisobutylene diol is a relatively nonpolar diol compared to other diols which may be used in forming PIB-PUR, such as polytetramethylene oxide diol and 1,4 butanediol. Thus, a suitable catalyst must be phase compatible with both the nonpolar polyisobutylene diol and the other, more polar diols used in forming PIB-PUR. A suitable catalyst must also promote comparable reaction rates between the diisocyanate and each of the diols in order to form PIB-PUR having residues from each of the diols substantially uniformly distributed along the polymer.

Tin(II) 2-ethylhexanoate (stannous octoate) has been employed for decades as a suitable catalyst for making PIB-PUR. The use of tin(II) 2-ethylhexanoate catalyst increases the reaction rate such that the reaction may be substantially completed within a few hours. Once the polymerization reaction is complete, the organometallic catalysts remains mixed into PIB-PUR, although it is not part of the polymer itself. Although the ligands of the organometallic catalyst may breakdown to some extent over time, the active metal cation remains and may continue to catalyze side reactions, resulting in degradation of the PIB-PUR over time.

It was surprisingly found that a tertiary amine, 2,6-dimethylpyridine, is also a suitable catalyst for making PIB-PUR. The 2,6-dimethylpyridine catalyst increases the polymerization reaction rate such that the reaction may be substantially completed within as little as five minutes. Unlike the organometallic catalysts, once the polymerization reaction is complete, a substantial portion of the 2,6-dimethylpyridine may be removed from the PIB-PUR by heating to an elevated temperature. As a result, less of the tertiary amine catalyst is available in the PIB-PUR to continue to catalyze damaging side reactions compared to the amount of active metal cations remaining in PIB-PUR catalyzed with an organometallic catalyst. Thus, PIB-PUR made with a tertiary amine catalyst, such as 2,6-dimethylpyridine, and free of an organometallic catalyst, may have a longer lifespan than PIB-PUR made with an organometallic catalyst, such as tin(II) 2-ethylhexanoate.

In addition to 2,6-dimethylpyridine, other tertiary amine catalysts suitable for use in making PIB-PUR may include 2-tert-butyl-1,1,3,3-tetramethylguanidine, 1,5-diazabicyclo[4.3.0]non-5-ene, 1,8-diazabicyclo[5.4.0]undec-7-ene, N,N-diisopropylmethylamine, N,N-dimethylcyclohexylamine, N,N,N',N',N"-pentamethyldiethylenetriamine, 4-methylmorpholine, 4-ethylmorpholine, 1-methylimidazole, N-ethyldiisopropylamine, 7-methyl-1,5,7-triazabicyclo[4.4.0]dec-5-ene, N,N,N,N-tetramethylethylenediamine, N,N,N'-trimethylethylenediamine, tributylamine, triethylamine, and tris[2-(dimethylamino)ethyl]amine. Some tertiary amine catalysts, such as trimethylamine, may be less suitable for use at the elevated temperatures used for polymerization because they have relatively high vapor pressures and may be driven from the PIB-PUR before the polymerization reaction is complete.

The PIB-PUR can be incorporated into medical devices which can be implanted or inserted into the body of a patient. Example medical devices may include, without limitation, vascular grafts, electrical leads, catheters, leadless cardiac pacemakers (LCP), pelvic floor repair support devices, shock coil coverings, covered stents including for intestine, esophageal and airway applications, urethral stents, internal feeding tube/balloon, embolics/bulking agents including mitral valve repair, structural heart applications including PFO, valve leaflets, and left atrial appendage, suture sleeves, breast implants, ophthalmic applications including intraocular lenses and glaucoma tubes, and spinal disc repair. Example electrical leads may include, without limitation, implantable electrical stimulation or diagnostic systems including neurostimulation systems such as spinal cord stimulation (SCS) systems, deep brain stimulation (DBS) systems, peripheral nerve stimulation (PNS) systems, gastric nerve stimulation systems, cochlear implant systems, and retinal implant systems, among others, and cardiac systems including implantable cardiac rhythm management (CRM) systems, implantable cardioverter-defibrillators (ICD's), and cardiac resynchronization and defibrillation (CRDT) devices, among others.

### EXAMPLES

The present invention is more particularly described in the following examples that are intended as illustrations only, since numerous modifications and variations will be apparent to those of skill in the art.

Polyisobutylene-polyurethane block copolymer including soft segments including at least one polyisobutylene diol residue and hard segments including at least one diisocyanate residue was prepared with a tertiary amine catalyst as follows. In a 1 liter resin kettle, 105.22 grams of polyisobutylene diol according to Formula I above, 71.72 grams of 4'-methylenediphenyl diisocyanate, and 56.88 grams of polytetramethylene oxide diol were dissolved in 541.15 grams of 2,6-dimethylpyridine. The 1 liter resin kettle was equipped with overhead mechanical stirring and was purged with dry nitrogen gas. The polyisobutylene diol had a number average molecular weight (Mn) of 1,932 grams/mole and the polytetramethylene oxide diol had a Mn of 1,000 grams/mole. The solution was stirred by the overhead mechanical stirrer under the flow of nitrogen gas at a temperature of 60 degrees Celsius while the reagents reacted for 2 hours. After the 2 hours, 15.78 grams of freshly distilled 1,4-butanediol was added to the solution by dropwise addition and the solution maintained at a temperature of 70 degrees Celsius while the reaction continued for an additional 2 hours. No organometallic catalyst, or any catalyst other than the 2,6-dimethylpyridine, was added to the reaction at any point. After the additional 2 hours, the solution was highly viscous solution containing the polyisobutylene-polyurethane block copolymer.

The resulting polyisobutylene-polyurethane block copolymer was characterized by gel permeation chromatography using a multi angle light scattering detector (GPC-MALLS). The copolymer exhibited a Mn of 109,000 grams/mole and a weight average molecular weight (Mw) of 274,300 grams/mole, indicating substantial completion of the reaction in 4 hours. In contrast, the inventors have not been able to substantially complete this synthesis without the use of catalyst. Thus, the tertiary amine 2,6-dimethylpyridine is demonstrated to be an effective catalyst for the production of polyisobutylene-polyurethane block copolymer including soft segments including at least one polyisobutylene diol residue and hard segments including at least one diisocyanate residue.

## Claims

1. A polymeric material comprising:
a polyisobutylene-polyurethane block copolymer including:
soft segments including at least one polyisobutylene diol residue; and
hard segments including at least one diisocyanate residue; and
a tertiary amine catalyst, wherein the tertiary amine catalyst includes 2,6-dimethylpyridine.

2. The polymeric material of claim 1, wherein the soft segments are present in the copolymer in an amount of about 40% to about 70% by weight of the copolymer, and the hard segments are present in the copolymer in an amount of about 30% to about 60% by weight of the copolymer.

3. The polymeric material of either of claims 1 or 2, wherein the polymeric material is free of an organometallic catalyst.

4. The polymeric material of any of claims 1-3, wherein the tertiary amine catalyst consists of 2,6-dimethylpyridine.

5. The polymeric material of any of claims 1-4, wherein the diisocyanate residue includes 4,4'-methylene diphenyl diisocyanate residue.

6. The polymeric material of any of claims 1-5, wherein the hard segments further include a chain extender residue.

7. The polymeric material of claim 6, wherein the chain extender residue is 1,4-butanediol residue.

8. The polymeric material of any of claims 1-7, wherein the soft segments further include at least one of a polyether diol residue, a polyester diol residue, and a polycarbonate diol residue.

9. A medical device comprising the polymeric material of any of claims 1-8.

10. A method of making a polymeric material, the method comprising:
heating a mixture of soft segment components and hard segment components to an elevated temperature, the soft segment components including at least one polyisobutylene diol, and the hard segment components including at least one diisocyanate; and
reacting the heated mixture of soft segment components and hard segment components by adding a tertiary amine catalyst to the mixture while maintaining the mixture at an elevated temperature, wherein the tertiary amine catalyst includes 2,6-dimethylpyridine.

11. The method of claim 10, wherein the soft segment components are present in the mixture in an amount of about 40% to about 70% by weight of the soft segment components and the hard segment components together, and the hard segment components are present in the mixture in an amount of about 30% to about 60% by weight of the soft segment components and the hard segment components together.

12. The method of either of claims 10 or 11, further including reacting a chain extender with the heated mixture and the tertiary amine catalyst.

13. The method of any of claims 10-12, wherein the tertiary amine catalyst consists of 2,6-dimethylpyridine.

14. The method of any of claims 10-13, wherein the polymeric material is free of an organometallic catalyst.

## Patentansprüche

1. Polymermaterial, umfassend:
ein Polyisobutylen-Polyurethan-Blockcopolymer, umfassend:
weiche Segmente, umfassend mindestens einen Polyisobutylendiolrest; und harte Segmente, umfassend mindestens einen Diisocyanatrest; und
einen Tertiäraminkatalysator, wobei der Tertiäraminkatalysator 2,6-Dimethylpyridin umfasst.

2. Polymermaterial nach Anspruch 1, wobei die weichen Segmente in dem Copolymer in einer Menge von etwa 40 % bis etwa 70 % Gewichtsanteilen des Copolymers vorliegen, und die harten Segmente in dem Copolymer in einer Menge von etwa 30 % bis etwa 60 % Gewichtsanteilen des Copolymers vorliegen.

3. Polymermaterial nach einem der Ansprüche 1 oder 2, wobei das Polymermaterial frei von einem organometallischen Katalysator ist.

4. Polymermaterial nach einem der Ansprüche 1-3, wobei der Tertiäraminkatalysator aus 2,6-Dimethylpyridin besteht.

5. Polymermaterial nach einem der Ansprüche 1-4, wobei der Diisocyanatrest einen 4,4'-Methylendiphenyldiisocyanatrest umfasst.

6. Polymermaterial nach einem der Ansprüche 1-5, wobei die harten Segmente ferner einen Kettenverlängererrest umfassen.

7. Polymermaterial nach Anspruch 6, wobei der Kettenverlängererrest ein 1,4-Butandiolrest ist.

8. Polymermaterial nach einem der Ansprüche 1-7, wobei die weichen Segmente ferner mindestens einen aus einem Polyetherdiolrest, einem Polyesterdiolrest und einem Polycarbonatdiolrest umfassen.

9. Medizinische Vorrichtung, umfassend das Polymermaterial nach einem der Ansprüche 1-8.

10. Verfahren zum Herstellen eines Polymermaterials, wobei das Verfahren umfasst:
Erwärmen eines Gemischs aus weichen Segmentkomponenten und harten Segmentkomponenten auf eine erhöhte Temperatur, wobei die weichen Segmentkomponenten mindestens ein Polyisobutylendiol umfassen und die harten Segmentkomponenten mindestens ein Diisocyanat umfassen; und
Reagieren des erwärmten Gemischs aus weichen Segmentkomponenten und harten Segmentkomponenten durch Hinzufügen eines Tertiäraminkatalysators zu dem Gemisch, während das Gemisch auf einer erhöhten Temperatur gehalten wird, wobei der Tertiäraminkatalysator 2,6-Dimethylpyridin umfasst.

11. Verfahren nach Anspruch 10, wobei die wobei die weichen Segmentkomponenten in dem Gemisch in einer Menge von etwa 40 % bis etwa 70 % Gewichtsanteilen der weichen Segmentkomponenten und der harten Segmentkomponenten gemeinsam vorliegen, und die harten Segmentkomponenten in dem Gemisch in einer Menge von etwa 30 % bis etwa 60 % Gewichtsanteilen der weichen Segmentkomponenten und der harten Segmentkomponenten gemeinsam vorliegen.

12. Verfahren nach einem der Ansprüche 10 oder 11, ferner umfassend das Reagieren eines Kettenverlängerers mit dem erwärmten Gemisch und dem Tertiäraminkatalysator.

13. Verfahren nach einem der Ansprüche 10-12, wobei der Tertiäraminkatalysator aus 2,6-Dimethylpyridin besteht.

14. Verfahren nach einem der Ansprüche 10-13, wobei das Polymermaterial frei von einem organometallischen Katalysator ist.

## Revendications

1. Matériau polymérique comprenant :
un copolymère bloc polyisobutylène-polyuréthane qui inclut :
des segments mous qui incluent au moins un résidu de polyisobutylène diol ; et des segments durs qui incluent au moins un résidu de diisocyanate ; et
un catalyseur d'amine tertiaire, dans lequel le catalyseur d'amine tertiaire inclut de la 2,6-diméthylpyridine.

2. Matériau polymérique selon la revendication 1, dans lequel les segments mous sont présents dans le copolymère selon une quantité qui va d'environ 40 % à environ 70 % en poids du copolymère, et les segments durs sont présents dans le copolymère selon une quantité qui va d'environ 30 % à environ 60 % en poids du copolymère.

3. Matériau polymérique selon l'une quelconque des revendications 1 et 2, dans lequel le matériau polymérique est exempt d'un catalyseur organométallique.

4. Matériau polymérique selon l'une quelconque des revendications 1 à 3, dans lequel le catalyseur d'amine tertiaire est constitué par de la 2,6-diméthylpyridine.

5. Matériau polymérique selon l'une quelconque des revendications 1 à 4, dans lequel le résidu de diisocyanate inclut un résidu de 4,4'-méthylène diphényl diisocyanate.

6. Matériau polymérique selon l'une quelconque des revendications 1 à 5, dans lequel les segments durs incluent en outre un résidu d'agent d'extension de chaîne.

7. Matériau polymérique selon la revendication 6, dans lequel le résidu d'agent d'extension de chaîne est un résidu de 1,4-butanediol.

8. Matériau polymérique selon l'une quelconque des revendications 1 à 7, dans lequel les segments mous incluent en outre au moins un résidu pris parmi un résidu de polyéther diol, un résidu de polyester diol et un résidu de polycarbonate diol.

9. Dispositif médical comprenant le matériau polymérique selon l'une quelconque des revendications 1 à 8.

10. Procédé de fabrication d'un matériau polymérique, le procédé comprenant :
le chauffage d'un mélange de composants à segments mous et de composants à segments durs jusqu'à une température élevée, les composants à segments mous incluant au moins un polyisobutylène diol, et les composants à segments durs incluant au moins un diisocyanate ; et
le fait de faire réagir le mélange chauffé de composants à segments mous et de composants à segments durs en ajoutant un catalyseur d'amine tertiaire au mélange tout en maintenant le mélange à une température élevée, dans lequel le catalyseur d'amine tertiaire inclut de la 2,6-diméthylpyridine.

11. Procédé selon la revendication 10, dans lequel les composants à segments mous sont présents dans le mélange selon une quantité qui va d'environ 40 % à environ 70 % en poids des composants à segments mous et des composants à segments durs en association, et les composants à segments durs sont présents dans le mélange selon une quantité qui va d'environ 30 % à environ 60 % en poids des composants à segments mous et des composants à segments durs en association.

12. Procédé selon l'une quelconque des revendications 10 et 11, incluant en outre le fait de faire réagir un agent d'extension de chaîne avec le mélange chauffé et le catalyseur d'amine tertiaire.

13. Procédé selon l'une quelconque des revendications 10 à 12, dans lequel le catalyseur d'amine tertiaire est constitué par de la 2,6-diméthylpyridine.

14. Procédé selon l'une quelconque des revendications 10 à 13, dans lequel le matériau polymérique est exempt d'un catalyseur organométallique.
